# FASCICULE DE BREVET EUROPEEN

(11) **EP 1 948 129 B1**
(45) Date de publication et mention de la délivrance du brevet: **01.09.2010**
(21) Numéro de dépôt: 06831350.1
(22) Date de dépôt: 17.11.2006
(51) Int. Cl.: A61K 8/97, A61Q 19/08

(54) **PROCEDE D'OBTENTION D'UN PRINCIPE ACTIF EXHAUSTEUR DE LA RESISTANCE MECANIQUE CUTANEE, PRINCIPE ACTIF ET COMPOSITIONS**
VERFAHREN ZUR GEWINNUNG EINES WIRKSTOFFS ZUR ERHÖHUNG DER MECHANISCHEN STÄRKE DER HAUT, WIRKSTOFF UND ZUSAMMENSETZUNGEN
PROCESS FOR OBTAINING AN ACTIVE INGREDIENT FOR ENHANCING THE MECHANICAL STRENGTH OF SKIN, ACTIVE INGREDIENT AND COMPOSITIONS

(30) Priorité: 18.11.2005 FR 0553497
(43) Date de publication de la demande: 30.07.2008
(73) Titulaire: Société Industrielle Limousine d'Application Biologique Dite SILAB, 19130 Objat (FR)
(72) Inventeur: PAUFIQUE, Jean, F-19130 Objat (FR)
(74) Mandataire: Fantin, Laurent
(86) Numéro de dépôt international: PCT/FR2006/051188
(87) Numéro de publication internationale: WO 2007/057615

(56) Documents cités:
- WO-A-02/28360
- WO-A-98/18342
- DE-A1- 4 301 010
- FR-A- 2 773 993
- FR-A- 2 802 418
- FR-A- 2 863 164

## Description

La présente demande se rapporte à un procédé d'obtention d'un principe actif issu de fibres et/ou de graines et/ou de son de seigle, pour augmenter la résistance mécanique de la peau et lutter notamment contre l'apparition des signes du vieillissement cutané.

L'invention concerne le principe actif susceptible d'être obtenu par ce procédé et les compositions cosmétiques associées.

Des principes actifs obtenus à partir d'un matériau végétal à utilisation cosmétique ou comme agent aromatisant alimentaire sont déjà connus dans l'art antérieure:
le document FR-A-2802418 décrit un principe actif cosmétique obtenu à partir de graines de blé, présentant un effet tenseur de la peau.
Le document WO-A-0228360 divulgue un principe actif cosmétique obtenu à partir de tourteau de céréales, présentant un effet tenseur immédiat de la peau.
Le document WO-A-9818342 décrit un procédé d'obtention d'un principe actif à partir d'un matériau végétal comme par example de fèves de soja, le lupine, le blé, la siégle, l'avoine. Le principe actif obtenu est utilisé comme agent parfumant alimentaire.

Dans nos sociétés, on accorde une grande importance au vieillissement cutané, en particulier aux aspects esthétiques et psychosociaux qui en découlent.

Le vieillissement cutané résulte de diverses altérations du derme provoquées par des facteurs à la fois génétiques et environnementaux. Il se manifeste notamment par la perte de la résistance mécanique et des propriétés liftantes du derme. La peau a alors tendance à s'étendre sous l'influence de son propre poids, provoquant ainsi des déformations de surface, la formation de rides et de plis disgracieux notamment au niveau des paupières et de la partie basse du massif facial.

Pour paraître plus jeunes et raffermir leur peau, beaucoup de gens souhaitent atténuer ces modifications physiques inesthétiques directement visibles.

C'est ce que propose le principe actif selon la présente invention en stimulant les équipements naturels cutanés impliqués dans la résistance mécanique de la peau et en renormalisant ses propriétés liftantes naturelles.

Le derme est un tissu conjonctif de soutien essentiellement constitué de fibroblastes et d'un réseau microfibrillaire de collagène, d'élastine et de protéoglycanes formant la matrice extracellulaire.

La matrice extracellulaire est le substrat d'adhésion des fibroblastes et le support mécanique du tissu. En cas de pression à la surface de la peau, les fibroblastes détectent les contraintes mécaniques transmises par des mécanorécepteurs, responsables de la jonction entre les fibroblastes et la matrice extracellulaire, et répondent notamment en stimulant la synthèse de collagène et en inhibant la production des métalloprotéinases.

Les mécanorécepteurs sont constitués d'intégrines, protéines de la membrane cellulaire dont la partie intracellulaire est associée à un complexe d'adhésion focale composé d'un ensemble de protéines de structure et de signalisation telle que la vinculine. Au niveau de ce complexe, sont attachées des fibres de stress dont l'alpha-smooth muscle actine (α-SMA), éléments rétractiles du cytosquelette du fibroblaste. Ces fibres, produites par les fibroblastes pour élever leurs propriétés rétractiles, assurent la maturation des mécanorécepteurs et permettent une transmission du signal mécanique plus efficace.

L'activité synergique des mécanorécepteurs et des fibres d'α-SMA assure l'équilibre perpétuel entre l'état de contraction et de relaxation cutanée et régule ainsi les propriétés liftantes naturelles de la peau.

Lors du vieillissement cutané, cet équilibre est perturbé conduisant à la perte des propriétés mécaniques du derme.

Les fibroblastes provenant de peaux âgées ou photoexposées montrent en particulier une diminution des intégrines α2β1 et de la vinculine, et donc une diminution de la formation des mécanorécepteurs, qui conduit à la perte du contact entre les fibroblastes et la matrice extracellulaire.

De fait, la présente invention a pour objectif de favoriser la formation des mécanorécepteurs en stimulant la synthèse des intégrines et de la vinculine, protéine marqueur des adhésions focales, et ainsi restaurer les capacités d'adhésion des fibroblastes.

En outre, les fibroblastes issus de peaux âgées sont aussi caractérisés par des fibres de stress courtes et désorganisées contrairement aux fibroblastes de peaux jeunes. Cette désorganisation du cytosquelette entraîne une réduction des forces rétractiles et par conséquent une résistance mécanique cutanée amoindrie.

C'est pourquoi la présente invention se propose également d'accroître la résistance mécanique de la peau en stimulant la synthèse des fibres d'α-SMA responsables de la génération des forces rétractiles des fibroblastes.

Le principe actif selon la présente invention restaure donc la résistance de la peau et ses propriétés liftantes naturelles à la fois :
- en favorisant la formation des mécanorécepteurs par stimulation de la synthèse de l'intégrine α2β1 et de la vinculine, et
- en augmentant la résistance mécanique de la peau par stimulation de la synthèse d'α-SMA.

Ainsi, en dopant les équipements naturels cutanés impliqués dans la résistance mécanique de la peau et en renormalisant ses propriétés liftantes naturelles, le principe actif selon l'invention raffermit le tissu cellulaire. Avantageusement, il permet de renforcer la résistance mécanique de la peau en augmentant la tonicité et la tension de la peau, et d'atténuer les rides et ridules notamment au niveau de la patte d'oie et du sillon nasogénien.

La présente invention est maintenant décrite en détail pour permettre de mieux appréhender les résultats obtenus regroupés en tableaux.

### I/ PROCEDE D'OBTENTION DU PRINCIPE ACTIF SELON L'INVENTION :

Le procédé selon la présente invention comprend la succession des étapes suivantes :
- solubilisation de fibres et/ou de graines et/ou de son de seigle dans l'eau,
- hydrolyse(s) enzymatique(s) simultanées ou successives,
- réparation des phases soluble et insoluble par filtration, centrifugation, décantation,
- traitement de la fraction active, et
- filtration stérilisante.

Selon un mode de réalisation préféré de l'invention, le procédé comprend la succession des étapes suivantes :
- solubilisation de graines et/ou de son de seigle dans l'eau,
- hydrolyse(s) enzymatique(s) simultanées ou successives,
- séparation des phases soluble et insoluble par filtration, centrifugation, décantation,
- traitement thermique,
- purification de la fraction active par filtration, et
- filtration stérilisante.

### II/CARACTERISATION DU PRINCIPE ACTIF SELON L'INVENTION

### II.1/ Matières sèches

On mesure le taux de matières sèches par passage à l'étuve à 105°C d'un échantillon de poids initial donné jusqu'à obtention d'un poids constant.

Le taux de matières sèches est compris entre 17 et 210 g/l, plus particulièrement entre 50 et 70 g/l.

### II.2/ Mesure du pH

Le pH mesuré par la méthode potentiométrique à température ambiante conduit à des valeurs comprises entre 6 et 9, plus particulièrement entre 7 et 8.

### II3/ Détermination de la teneur en sucres totaux

On utilise la méthode de DUBOIS (DUBOIS M. & al [1956], Analytical chemistry, 28, n°3 p 350-356).

En présence d'acide sulfurique concentré et de phénol, les sucres réducteurs donnent un composé jaune-orangé.

A partir d'une gamme étalon, on peut déterminer le taux de sucres totaux d'un échantillon.

Le taux de sucres totaux du principe actif selon la présente invention est de 14 à 195 g/l, préférentiellement de 45 à 65 g/l.

### II.4/ Caractérisation des carbohydrates

### a. Dosage de sucres simples

Le taux de sucres simples est réparti en 96,4% de glucose, en 2,6% de xylose et en 1,0% d'arabinose.

### b. Degré de polymérisation

Le tableau ci-dessous montre que la fraction glucidique du principe actif selon la présente invention comprend du glucose, du xylose et de l'arabinose essentiellement sous forme de disaccharides, pentasaccharides et d'oligosaccharides.

| | Degré de polymérisation | Taux de carbohydrates |
|---|---|---|
| Monosaccharides | 1 | 4,8% |
| Disaccharides | 2 | 25,0% |
| Oligosaccharides | 3 | 8,5% |
| Oligosaccharides | 4 | 3,9% |
| Oligosaccharides | 5 | 19,1% |
| Oligosaccharides | 6 | 7,0% |
| Oligosaccharides et polysaccharides | ≥ 7 | 27,6% |

### II.5/ Caractérisation des composés phénoliques

La caractérisation et la quantification des composés phénoliques du principe actif obtenu selon l'invention sont réalisées par HPLC, (High Performance Liquid Chromatography).

Le chromatogramme du principe actif obtenu selon l'invention montre la présence de composés hydroxybenzoïques et de composés hydroxycinnamiques, les proportions étant sensiblement de l'ordre des valeurs données dans le tableau suivent :

| **Composés phénoliques identifiés** | **Concentration** |
|---|---|
| Hydroxybenzoïques | **16,1%** |
| Hydroxycinnamiques | **83,9%** dont 71,4% d'acide férulique et 10,1% d'acide p-coumarique |

### II.6/ Identification de la fraction active

Dans le but d'identifier la ou les fractions actives, on fractionne les sucres du principe actif obtenu selon l'invention par chromatographie gel filtration.

L'efficacité de la fraction obtenue est évaluée par l'expression des ARNm de la vinculine par PCR (Polymerase Chain Reaction ou Amplification en chaîne par Polymérase) quantitative.

L'analyse des résultats montre que l'efficacité du principe actif obtenu selon l'invention réside dans une fraction oligosaccharidique riche en arabinoxylane et en glucose.

### III/ Evaluation de l'effet du principe actif

### III.1/ Evaluation de l'effet sur la synthèse des protéines du mécanorécepteur

Cette étude a pour objectif d'évaluer l'effet du principe actif obtenu selon l'invention sur l'expression de protéines du mécanorécepteur, à savoir :
- les intégrines de type α2β1, et
- la vinculine, marqueur des adhésions focales.

L'étude est réalisée par PCR quantitative sur des fibroblastes humains normaux, comparé à un modèle de fibroblastes humains vieillis.

A J1, les fibroblastes humains normaux et vieillis sont ensemencés et incubés à 37°C.

A J3, les cellules sont traitées. Elles sont cultivées en présence du principe actif obtenu selon l'invention dosé à 0,25% ou de TGF-β1 à 1ng/ml, molécule de référence.

A J4, les cellules sont récupérées et les ARN totaux sont extraits. Les ARN sont reverses-transcripts et les ADN complémentaires obtenus sont analysés par la technique de PCR quantitative.

### a. Effet sur la synthèse des intégrines α2β1 sur fibroblastes humains normaux et fibroblastes humains vieillis

Les résultats obtenus concernant l'expression des ARN messagers (ARNm) des intégrines α2β1 sont exprimés en pourcentage dans le tableau suivant :

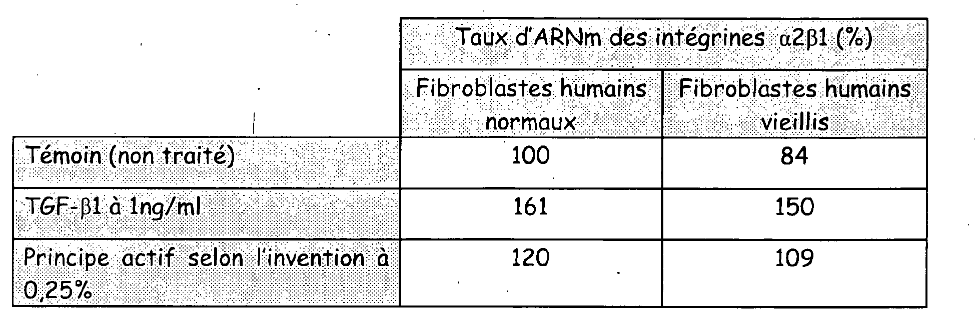

On constate que l'expression des ARNm des intégrines α2β1 par les fibroblastes vieillis est réduite de 16% par rapport aux fibroblastes humains normaux.

Les résultats de l'étude montrent que le principe actif selon l'invention dosé à 0,25% augmente de 20% l'expression des ARNm des intégrines α2β1 par les fibroblastes normaux et restaure l'expression des ARNm des intégrines α2β1 par les fibroblastes vieillis.

### b. Effet sur la synthèse de la vinculine sur fibroblastes humains normaux et fibroblastes humains vieillis

Les résultats obtenus concernant l'expression des ARNm de la vinculine sont exprimés en pourcentage dans le tableau suivant :

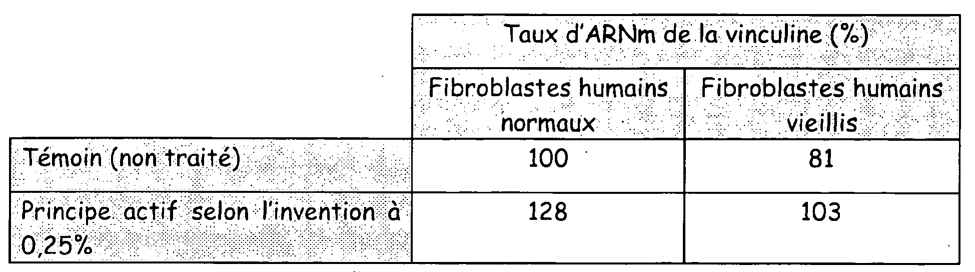

On constate que l'expression des ARNm de la vinculine par les fibroblastes vieillis est réduite de 19% par rapport aux fibroblastes humains normaux.

Le principe actif selon l'invention dosé à 0,25% augmente de 28% l'expression des ARNm de la vinculine par les fibroblastes normaux et restaure l'expression des ARNm de la vinculine par les fibroblastes vieillis.

Le principe actif selon l'invention favorise donc la formation des mécanorécepteurs en stimulant la synthèse de l'intégrine α2β1 et de la vinculine, et participe ainsi à l'augmentation de la résistance de la peau et de ses propriétés liftantes naturelles.

### III.2/Evaluation de l'effet sur l'expression de l'alpha smooth muscle actine

L'objectif de l'étude est d'évaluer l'effet du principe actif obtenu selon l'invention sur l'expression de l'alpha-smooth muscle actine (α-SMA), fibre de stress impliquée dans les propriétés rétractiles des fibroblastes. Cette étude est réalisée sur des fibroblastes humains normaux comparés à un modèle de fibroblastes humains vieillis.

### a. Etude sur fibroblastes humains normaux et fibroblastes humains vieillis par spectrofluorimétrie

Le protocole opératoire est le suivant :
A J1, les fibroblastes humains normaux et vieillis sont ensemencés et incubés à 37°C.
A J3, les cellules sont traitées. Elles sont cultivées en présence du principe actif selon l'invention à 0,25% ou 0,50% ou de TGF-β1 à 1ng/ml.
A J5, les cellules sont traitées comme à J3.
A J8, on réalise l'immunomarquage notamment avec un anticorps primaire anti-α-SMA. La fluorescence est quantifiée à l'aide d'un fluorimètre.

Les résultats obtenus, exprimés en pourcentage d'α-SMA exprimés, sont présentés dans le tableau suivant :

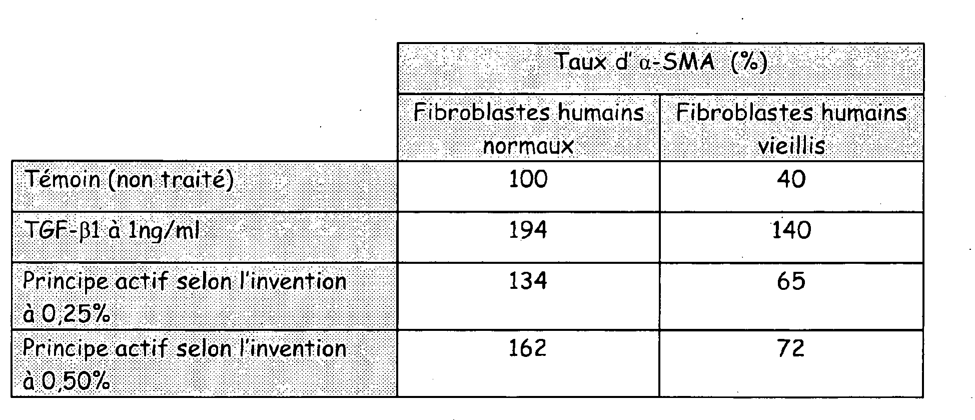

Ces résultats montrent que le principe actif obtenu selon l'invention testé à 0,50% sur fibroblastes normaux augmente le taux des α-SMA de 62% par rapport au témoin, et stimule le taux des α-SMA sur fibroblastes vieillis.

### b. Etude sur fibroblastes humains normaux et fibroblastes humains vieillis par immunocytologie

Le protocole opératoire est le suivant :
A J1, les fibroblastes humains sont ensemencés dans un milieu de culture complet.
A J3, les cellules sont traitées. Les fibroblastes humains normaux et vieillis sont traités avec le principe actif selon l'invention à 0,10% ou avec le TGF-β1 à 0,50 ng/ml dans du milieu de culture complet.
A J5, les cellules sont traitées comme à J3.
A J8, on réalise le marquage immunocytologique des α-SMA, puis on réalise un immunomarquage à l'aide notamment d'un anticorps primaire anti-α-SMA.

Les résultats sont visualisés sur un microscope couplé à un système d'analyse d'images. Ces résultats immunohistochimiques étant qualitatifs, 4 niveaux d'expression des α-SMA ont été définis :
- très faible détection d'immunoréactivité +
- faible détection d'immunoréactivité ++
- moyenne détection d'immunoréactivité +++
- forte détection d'immunoréactivité ++++

Les résultats obtenus sont présentés dans le tableau qui suit :

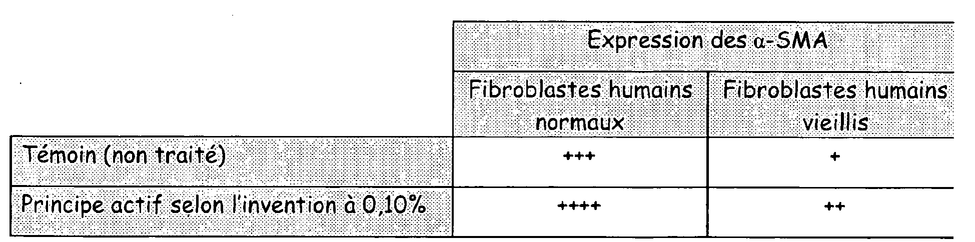

On constate que le principe actif obtenu selon l'invention dosé à 0,10% augmente l'expression des α-SMA sur fibroblastes.

Le principe actif obtenu selon l'invention augmente donc la résistance mécanique de la peau en stimulant la synthèse d'α-SMA.

### III.3/Evaluation de l'effet sur l'expression de collagène I

Cette étude a pour objectif d'évaluer l'effet du principe actif obtenu selon l'invention sur la synthèse de collagène I sur des fibroblastes.

L'étude est réalisée par PCR quantitative sur des fibroblastes humains normaux comparés à un modèle de fibroblastes humains vieillis.

A J1, les fibroblastes humains normaux et vieillis sont ensemencés à 37°C. A J3, les cellules sont traitées. Elles sont cultivées en présence du principe actif obtenu selon l'invention dosé à 0,10% ou 0,25%.

A J4, les cellules sont récupérées et les ARN totaux sont extraits.

Les ARN sont reverses-transcripts et les ADN complémentaires obtenus sont analysés par la technique de PCR quantitative.

Les résultats obtenus concernant l'expression des ARN messagers (ARNm) de collagène I sont exprimés en pourcentage dans le tableau suivant :

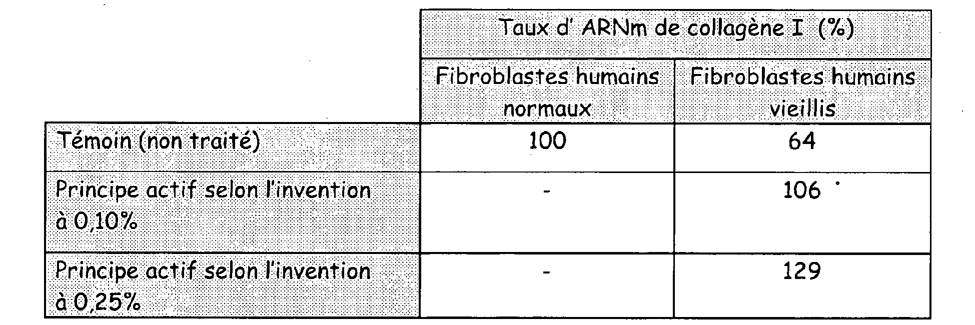

On constate que l'expression des ARNm de collagène I par les fibroblastes vieillis est réduite de 36% par rapport aux fibroblastes humains normaux. Les résultats de l'étude montrent que le principe actif selon l'invention dosé à 0,25% augmente de 29% l'expression des ARNm de collagène I par les fibroblastes vieillis.

### III.4/Evaluation de l'effet sur les propriétés biomécaniques de la peau

Le but de cette étude est d'évaluer l'efficacité du principe actif obtenu selon l'invention formulé à 4% en émulsion contre placebo sur les propriétés biomécaniques de la peau.

L'étude est réalisée sur 20 volontaires sains de sexe féminin d'âge compris entre 39 et 70 ans.

Les mesures sont réalisées au niveau du visage à l'aide d'un cutomètre. Le cutomètre est un appareil de mesure qui aspire la peau et permet de calculer divers paramètres caractéristiques des propriétés mécaniques cutanées :
- La tonicité de la peau est appréciée par le paramètre X qui correspond à la tonicité ou rétractation élastique ; si X diminue, la tonicité augmente.
- L'effet tenseur est apprécié par les paramètres Uf et Ue ; si Uf diminue, la peau est moins extensible donc plus tendue et si Ue diminue la peau est moins souple donc plus tendue également.

L'étude est réalisée selon le protocole opératoire qui suit.

A J0, on détermine deux zones cutanées symétriques au niveau du visage de chaque volontaire, une destinée au placebo, l'autre à l'émulsion contenant le principe actif obtenu, et on mesure les propriétés biomécaniques de la peau sur ces deux zones.

Entre J0 et J27, l'actif et le placebo sont appliqués biquotidiennement.

A J28, on mesure les propriétés mécaniques de la peau sur les mêmes zones qu'à J0.

Entre J28 et J55, l'actif et le placebo sont appliqués biquotidiennement. A J56, on mesure les propriétés mécaniques de la peau sur les zones étudiées.

### a. Tonicité de la peau

Les résultats obtenus pour le paramètre -X sont les suivants :

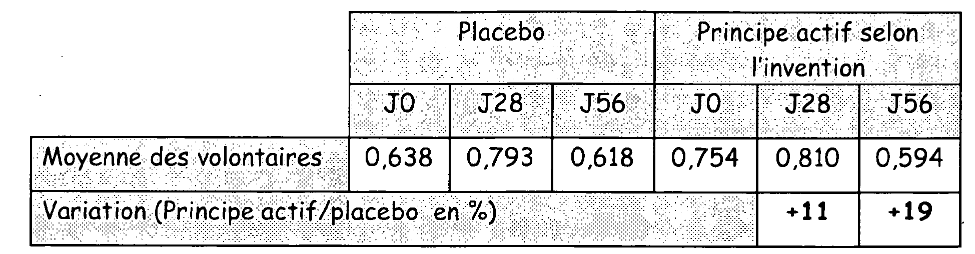

Ces résultats montrent qu'après 56 jours d'applications biquotidiennes et en comparaison au placebo, le principe actif obtenu selon l'invention augmente le paramètre -X caractéristique de la tonicité de la peau de 19%.

### b. Tension de la peau

Les résultats pour le paramètre -Uf sont exprimés dans le tableau qui suit :

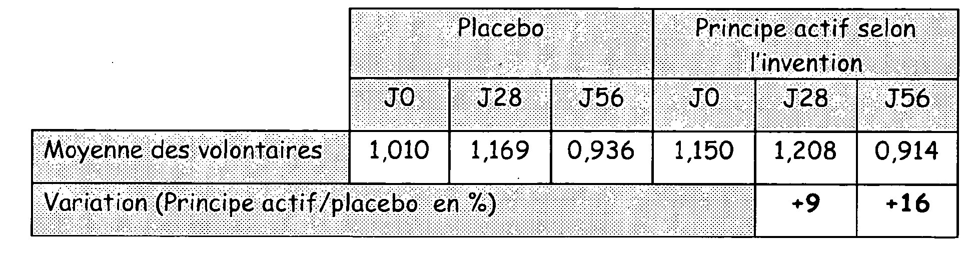

Concernant le paramètre -Ue, les résultats sont les suivants :

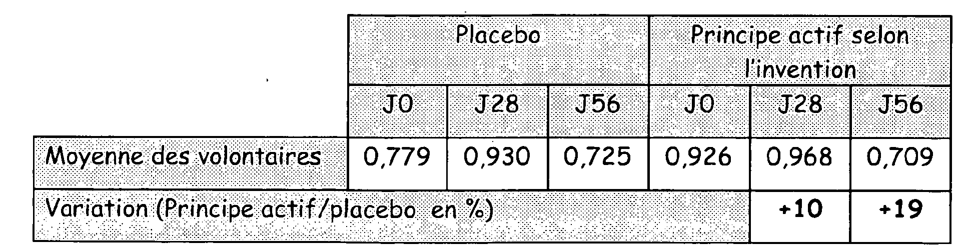

On constate que le principe actif obtenu selon l'invention testé à 4% en émulsion augmente les paramètres -Uf de 16% et -Ue de 19%, paramètres représentatifs de la tension de la peau.

Le principe actif obtenu selon l'invention améliore donc les propriétés biomécaniques de la peau : il augmente la tonicité et la tension cutanée.

### III.5/Etude des propriétés anti-rides

Cette étude a pour objectif de quantifier l'efficacité anti-rides du principe actif obtenu selon l'invention formulé à 4% en émulsion contre placebo.

Elle est réalisée sur 20 volontaires sains de sexe féminin d'âge compris entre 39 et 70 ans.

L'efficacité anti-rides est mesurée par l'intermédiaire d'empreintes siliconées réalisées au niveau des pattes d'oie ou du sillon naso-génien des volontaires. L'analyse de ces empreintes à l'aide d'un profilomètre muni d'un analyseur d'images permet d'obtenir trois paramètres : le nombre de rides, la surface totale ridée et la longueur totale des rides.

### a. Au niveau des pattes d'oie

L'étude est réalisée selon le protocole ci-dessous.

A J0, on détermine deux zones cutanées symétriques au niveau des pattes d'oie, une destinée à être traitée par le placebo, l'autre par le principe actif, et on prend les empreintes sur ces deux zones.

Entre J0 et J27, le principe actif et le placebo sont appliqués deux fois par jour.

A J28, les empreintes sont prises sur les deux zones étudiées.

Entre J28 et J55, le principe actif et le placebo sont appliqués deux fois par jour.

A J56, les empreintes sont prises sur les deux zones étudiées.

Les résultats obtenus pour le principe actif par rapport à ceux obtenus pour le placebo sont exprimés en pourcentage dans le tableau qui suit :

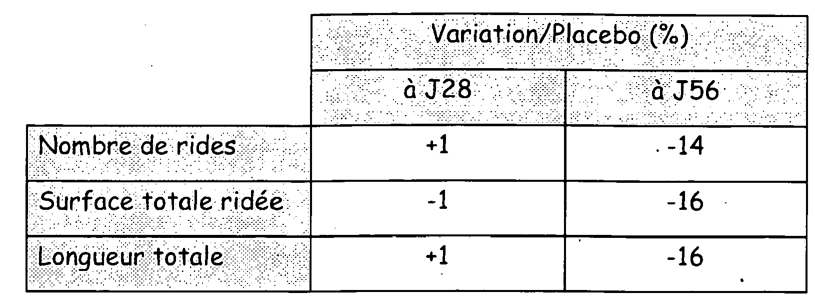

On constate qu'après 56 jours d'applications biquotidiennes en comparaison du placebo, le principe actif obtenu selon l'invention formulé à 4% en émulsion diminue à la fois le nombre de rides, la surface totale ridée et la longueur totale des rides.

### b. Au niveau du sillon noso-génien

L'étude est réalisée selon le protocole opératoire qui suit.

A J0, on détermine deux zones cutanées symétriques au niveau du sillon naso-génien, une destinée à être traitée par le placebo, l'autre par le principe actif, et on prend les empreintes sur ces deux zones.

Entre J0 et J27, le principe actif et le placebo sont appliqués deux fois par jour.

A J28, les empreintes sont prises sur les deux zones étudiées.

Entre J28 et J55, le principe actif et le placebo sont appliqués deux fois par jour.

A J56, les empreintes sont prises sur les deux zones étudiées.

Les résultats obtenus pour le principe actif en comparaison de ceux obtenus pour le placebo sont exprimés en pourcentage dans le tableau suivant :

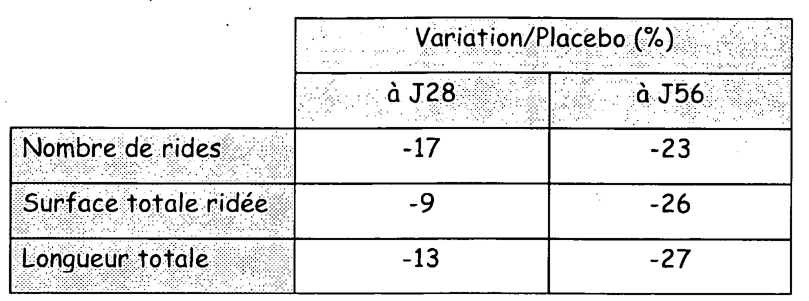

On constate qu'après 56 jours d'applications biquotidiennes en comparaison au placebo, le principe actif obtenu selon l'invention formulé à 4% en émulsion diminue le nombre de rides de 23% la surface totale ridée de 26% et la longueur totale des rides de 27%.

Le principe actif obtenu selon l'invention a donc des propriétés liftantes et un effet anti-rides.

### III.6/Etude des propriétés lissantes

Cette étude a pour objectif de quantifier l'efficacité lissante du principe actif obtenu selon l'invention formulé à 4% en émulsion contre placebo. L'étude est réalisée sur 20 volontaires sains de sexe féminin d'âge compris entre 39 et 70 ans.

L'efficacité lissante est mesurée par l'intermédiaire d'empreintes siliconées réalisées au niveau des pattes d'oie des volontaires. L'analyse de ces empreintes à l'aide d'un profilomètre muni d'un analyseur d'images permet d'obtenir deux paramètres : un indice Ra de rugosité moyenne, et un indice Rz de rugosité maximale.

L'étude est réalisée selon le protocole ci-dessous.

A J0, on détermine deux zones cutanées symétriques au niveau des pattes d'oie, une destinée à être traitée par le placebo, l'autre par le principe actif, et on prend les empreintes sur ces deux zones.

Entre J0 et J27, le principe actif et le placebo sont appliqués deux fois par jour.

A J28, les empreintes sont prises sur les deux zones étudiées. Entre J28 et J55, le principe actif et le placebo sont appliqués deux fois par jour.

A J56, les empreintes sont prises sur les deux zones étudiées.

Les résultats obtenus pour le principe actif par rapport à ceux obtenus pour le placebo sont exprimés en pourcentage dans le tableau qui suit :

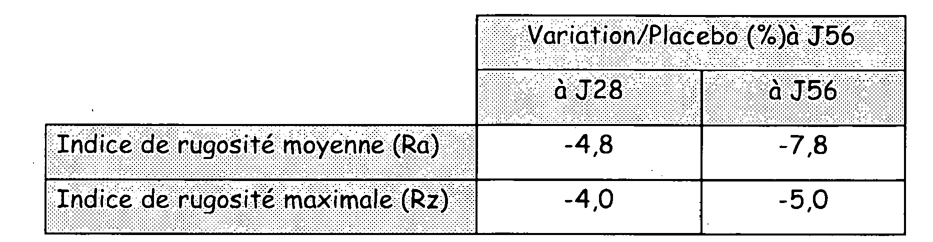

On constate qu'après 56 jours d'applications biquotidiennes en comparaison au placebo, le principe actif obtenu selon l'invention formulé à 4% en émulsion diminue les indices de rugosité moyenne et maximale.

Le principe actif obtenu selon l'invention présente donc un effet lissant.

### IV/ COMPOSITION COSMETIQUE INCLUANT LE PRINCIPE ACTIF SELON L'INVENTION :

La présente invention couvre aussi les compositions cosmétiques incluant le principe actif selon la présente invention dans différentes formes galéniques, notamment gel, solution, émulsion, crème....

Il convient alors d'analyser la stabilité des formes galéniques incluant le principe actif selon l'invention, ceci dans des proportions comprises entre 1 et 5 %.

La stabilité est caractérisée par une absence de précipitation de l'actif, une absence de crémage et une absence de déphasage.

On peut citer des formulations ayant montré une stabilité physique incluant 5% de principe actif selon l'invention.

| | |
|---|---|
| Gel limpide : | - Carbopol : 0,5% avec Triéthanolamine : qsp pH=6,5 - Phénonip : 0,7% - Principe actif 5,0% - Eau : 93,8% |
| Gel opaque : | - Sépigel 305 : 2,0% - Phénonip : 0,7% - Principe actif : 5,0% - Eau : 92,3% |
| Gel émulsionné : | - Montanov 202 : 3,0% - Isopropyl palmitate : 12,0% - Phénonip : 0,7% - Viscolam AT 64 : 2,0% - Principe actif : 5,0% - Eau : 77,3% |
| | |
| Emulsion non ionique : | - Montanov 202 : 3,0% - Simulsol 165 : 2,0% - Isopropyl palmitate : 20,0% - Phénonip : 0,7% - Principe actif : 5,0% - Eau : 69,3% |
| Emulsion anionique : | - Acide stéarique : 7,0% - Triethanolamine : 3,5% - Isopropyl palmitate : 20,0% - Phénonip : 0,7% - Principe actif 5,0% - Eau : 63,8% |
| Emulsion cationique : | - Quaternium-82 : 5,0% - Alcool cétylique : 3,0% - Isopropyl palmitate : 15,0% - Phénonip : 0,7% - Principe actif 5,0% - Eau : 71,3% |

De plus, des tests ont montré la compatibilité du principe actif avec les matières premières utilisées eh cosmétique.

## Revendications

1. Principe actif obtenu par la mise en oeuvre d'un procédé comprenant les étapes suivantes :
- solubilisation de fibres et/ou de graines et/ou de son de seigle dans l'eau,
- hydrolyse(s) enzymatique(s) simultanées ou successives,
- séparation des phases soluble et insoluble par filtration, centrifugation, décantation,
- traitement de la fraction active, et
- filtration stérilisante,
**caractérisé en ce qu'**il présente les paramètres suivants :
- taux de matières sèches compris entre 17 et 210 g/l,
- pH compris entre 6,0 et 9,0, et
- teneur en sucres totaux comprise entre 14 et 195 g/l.

2. Principe actif selon la revendication 1, **caractérisé par** les paramètres suivants :
- taux de matières sèches compris entre 50 et 70 g/l,
- pH compris entre 7,0 et 8,0,
- teneur en en sucres totaux comprise entre 45 et 65g/l,
- présence de carbohydrates sous forme de glucose, de xylose et d'arabinose, et
- présence de composés phénoliques sous forme d'hydroxybenzoïques et d'hydroxycinnamique.

3. Utilisation du principe actif selon l'une des revendication 1 ou 2, pour incorporation à une composition cosmétique, **caractérisée en ce qu'**il présente un effet exhausteur de la résistance mécanique cutanée.

4. Utilisation du principe actif selon l'une des revendications 1 ou 2, pour incorporation à une composition cosmétique, **caractérisée en ce qu'**il augmente la tension et la tonicité de la peau.

5. Utilisation du principe actif selon l'une des revendications 1 ou 2, pour incorporation à une composition cosmétique, **caractérisée en ce qu'**il présente un effet anti-rides.

6. Utilisation du principe actif selon l'une des revendications 1 ou 2, pour incorporation à une composition cosmétique, **caractérisée en ce qu'**il favorise la formation des mécanorécepteurs des fibroblastes du derme.

7. Utilisation du principe actif selon l'une des revendications 1 ou 2, pour incorporation à une composition cosmétique, **caractérisée en ce qu'**il stimule la synthèse des fibres d'alpha- smooth actine.

8. Composition cosmétique incluant le principe actif selon l'une des revendications 1 ou 2, **caractérisée en ce qu'**elle est constituée d'un gel limpide, d'un gel opaque, d'un gel émulsionné, d'une émulsion non ionique, d'une émulsion anionique ou d'une émulsion cationique.

## Claims

1. Active ingredient that is obtained by the implementation of a process comprising the following stages:
- Solubilization of rye fibers and/or seeds and/or bran in water,
- Simultaneous or successive enzymatic hydrolysis or hydrolyses,
- Separation of soluble and insoluble phases by filtration, centrifuging, decanting,
- Treatment of the active fraction, and
- Sterilizing filtration,
**characterized by** the following parameters:
- Level of dry materials of between 17 and 210 g/l,
- pH of between 6.0 and 9.0, and
- Total sugar content of between 14 and 195 g/l.

2. Active ingredient according to claim 1, **characterized by** the following, parameters:
- Level of dry materials of between 50 and 70 g/l,
- pH of between 7.0 and 8.0,
- Protein content of between 45 and 65 g/l,
- Presence of carbohydrates in the form of glucose, xylose and arabinose, and
- Presence of phenolic compounds in the form of hydroxybenzoic and hydroxycinnamic compounds.

3. Use of the active ingredient according to one of claims 1 or 2, for incorporation with a cosmetic composition, wherein it has an effect of enhancing cutaneous mechanical strength.

4. Use of the active ingredient according to one of claims 1 or 2, for incorporation with a cosmetic composition, wherein it increases the tension and the tonicity of the skin.

5. Use of the active ingredient according to one of claims 1 or 2, for incorporation with a cosmetic composition, wherein it has an anti-wrinkle effect.

6. Use of the active ingredient according to one of claims 1 or 2, for incorporation with a cosmetic composition, wherein it promotes the formation of the mechanoreceptors of the fibroblasts of the dermis.

7. Use of the active ingredient according to one of claims 1 or 2, for incorporation with a cosmetic composition, wherein it stimulates the synthesis of the alpha-smooth actin fibers.

8. Cosmetic composition that includes the active ingredient according to one of claims 1 or 2, wherein it consists of a clear gel, an opaque gel, an emulsified gel, a non-ionic emulsion, an anionic emulsion or a cationic emulsion.

## Patentansprüche

1. Wirkstoff, der mittels Durchführung eines Verfahrens erhalten wird, das die folgenden Schritte umfasst:
- Solubilisieren von Roggenfasern und/oder -samen und/oder -kleine in Wasser,
- gleichzeitige oder aufeinanderfolgende enzymatische Hydrolyse(n),
- Trennen der löslichen und der unlöslichen Phase durch Filtrieren, Zentrifugieren, Dekantieren,
- Behandeln der aktiven Fraktion und
- Sterilfiltration,
**dadurch gekennzeichnet, dass** er die folgenden Parameter aufweist:
- Trockenmassegehalt zwischen 17 und 210 g/l,
- pH-Wert zwischen 6,0 und 9,0 und
- Gesamtzuckergehalt zwischen 14 und 195 g/l.

2. Wirkstoff nach Anspruch 1, **gekennzeichnet durch** die folgenden Parameter:
- Trockenmassegehalt zwischen 50 und 70 g/l,
- pH-Wert zwischen 7,0 und 8,0,
- Gesamtzuckergehalt zwischen 45 und 65 g/l,
- Vorliegen von Kohlenhydraten in Form von Glucose, Xylose und Arabinose und
- Vorliegen phenolischer Verbindungen in Form von Hydroxybenzoe- und Hydroxyzimtsäuren.

3. Verwendung des Wirkstoffs nach einem der Ansprüche 1 oder 2, zum Einbringen in eine kosmetische Zusammensetzung, **dadurch gekennzeichnet, dass** er eine die mechanische Festigkeit der Haut stärkende Wirkung aufweist.

4. Verwendung des Wirkstoffs nach einem der Ansprüche 1 oder 2, zum Einbringen in eine kosmetische Zusammensetzung, **dadurch gekennzeichnet, dass** er die Spannung und die Spannkraft der Haut erhöht.

5. Verwendung des Wirkstoffs nach einem der Ansprüche 1 oder 2, zum Einbringen in eine kosmetische Zusammensetzung, **dadurch gekennzeichnet, dass** er eine Anti-Falten-Wirkung aufweist.

6. Verwendung des Wirkstoffs nach einem der Ansprüche 1 oder 2, zum Einbringen in eine kosmetische Zusammensetzung, **dadurch gekennzeichnet, dass** er die Bildung der Mechanorezeptoren der Fibroblasten der Dermis begünstigt.

7. Verwendung des Wirkstoffs nach einem der Ansprüche 1 oder 2, zum Einbringen in eine kosmetische Zusammensetzung, **dadurch gekennzeichnet, dass** er die Synthese der Alpha-Smooth-Actin-Fasern stimuliert.

8. Kosmetische Zusammensetzung, die den Wirkstoff nach einem der Ansprüche 1 oder 2 enthält, **dadurch gekennzeichnet, dass** sie von einem durchsichtigen Gel, einem opaken Gel, einem emulgierten Gel, einer nichtionischen Emulsion, einer anionischen Emulsion oder einer kationischen Emulsion gebildet ist.
